# EUROPEAN PATENT APPLICATION

(11) **EP 2 564 832 A1**
(43) Date of publication of application: **06.03.2013**
(21) Application number: 11179139.8
(22) Date of filing: 29.08.2011
(51) Int. Cl.: A61K 9/14, A61K 31/426, A61K 31/513

(54) **Solid dosage form of HIV protease inhibitors**

(71) Applicant: Hexal AG, 83607 Holzkirchen (DE)
(72) Inventor: Rother, Patrick, D-83607 Holzkirchen (DE); Fischer, Diana, D-83607 Holzkirchen (DE); Täubrich, Theresa, D-83607 Holzkirchen (DE); Oppelt, Renate, D-83607 Holzkirchen (DE)
(74) Representative: Gravert, Maike

(57) **Abstract**

A pharmaceutical dosage form suitable for oral administration comprising a solid dispersion of at least one HIV protease inhibitor, at least one first pharmaceutically acceptable water-soluble polymer (A), at least one second pharmaceutically acceptable water-soluble polymer (B), and optionally at least one pharmaceutically acceptable surfactant,wherein the at least one first pharmaceutically acceptable water-soluble polymer (A) has a glass transition temperature (Tg) of at least 50°C and is present in an amount of 10% to 49% by weight of the dosage form and is not polyvinylpyrrolidone (PVP) or hydroxypropyl methylcellulose (HPMC), and wherein the at least one second pharmaceutically acceptable water-soluble polymer (B) has a Tg of less than 50°C.

## Description

The present invention is directed to a solid pharmaceutical dosage form, preferably for oral administration, comprising at least one HIV protease inhibitor.

Acquired immunodeficiency syndrome (AIDS) is caused by a virus known i.a. as T-lymphocyte virus III (HTLV-111), lymphadenopathy- associated virus (LAV), AIDS-related virus (ARV) or human immunodeficiency virus (HIV), whereas two distinct families, HIV-1 and HIV-2 have been identified.

One of the critical pathways in retroviral life cycle is polyprotein precursors processing by aspartic protease. With the HIV virus the gag-pol protein is processed by HIV protease for instance. The correct processing of the precursor polyproteins by aspartic protease is required for the assembly of infectious virions, making the aspartic protease an promising therapeutic antiviral target. Thus, for HIV treatment, the HIV protease is an attractive target.

Whether or not an oral dosage form of a pharmaceutical agent is potentially useful for therapeutic purposes is the bioavailability thereof as observed after oral administration. Various factors may affect the bioavailability of an orally administered drug, including aqueous solubility, drug absorption throughout the gastrointestinal tract, dosage strength and first pass effect. Aqueous solubility is one of the most important of these factors.

One characteristic of HIV protease inhibiting compounds is their inherent poor aqueous solubility.

For several reasons, such as in particular patient compliance and taste masking, a solid dosage form is usually preferred over a liquid dosage form. In most circumstances however, oral solid dosage forms of a drug exhibit lower bioavailability than the same drug's oral solutions.

Attempts have been made to improve bioavailability of drugs in solid dosage forms by forming solid solutions of the drug. The term "solid solution" refers to a system in solid state wherein the drug is molecularly dispersed throughout a matrix, such that the system is chemically and physically uniform or homogenous throughout. Solid solutions are preferred physical systems because of the components readily forming liquid solutions therein when contacted with a liquid medium such as gastric juice. The ease of dissolution may be attributed at least in part to the fact that the energy required for dissolution of the components from a solid solution is less than that required for the dissolution of the components from a crystalline or microcrystalline solid phase. If, however, the drug absorption in the gastrointestinal tract is slow the drug released from the solid solution may result in a high supersaturation and subsequently precipitate in the aqueous gastrointestinal fluids.

A continuing need for the development of improved oral solid dosage forms for HIV protease inhibitors which have suitable oral bioavailability and stability and which do not necessitate high vehicle volumes is observed.

The present invention provides a pharmaceutical dosage form suitable for oral administration, comprising a solid dispersion of at least one HIV protease inhibitor in at least one first pharmaceutically acceptable water-soluble polymer (A) and at least one second pharmaceutically acceptable water-soluble polymer (B) and optionally at least one pharmaceutically acceptable surfactant.

The at least one first pharmaceutically acceptable water-soluble polymer (A) has a glass transition temperature (Tg) of at least 50°C and is present in an amount of 10 to 49% by weight, the weight being defined relative to the weight of the dosage form. The at least one first pharmaceutically acceptable water-soluble polymer (A) is not PVP or HPMC.

Preferably the at least one first water-soluble polymer (A) has a Tg of 50°C to 250°C, more preferably of at least 80°C to about 225°C.

The at least one second pharmaceutically acceptable water-soluble polymer (B) which is in each case different from polymer (A), has a Tg of less than 50°C, preferably less than 40°C.

The pharmaceutical dosage form of the invention preferably comprises the least one HIV protease inhibitor as a solid or glassy solution.

Glass transition temperature as used herein is the temperature at which reversible transition in amorphous materials from a hard and relatively brittle state into a molten or rubber-like state and vice versa occurs. Methods for determining glass transition temperature Tg values of the organic polymers are described in "Introduction to Physical Polymer Science", 2nd Edition by L. H. Sperling, published by John Wiley & Sons, Inc., 1992. The Tg value can be calculated as the weighted sum of the Tg values for homopolymers derived from each of the individual monomers, i.e., that make up the polymer: Tg = Σ Wᵢ Xᵢ, where W is the weight percent of monomer i in the organic polymer, and X is the Tg value for the homopolymer derived from monomer i. Tg values for the homopolymers may be taken from "Polymer Handbook", 2nd Edition by J. Brandrup and E. H. Immergut, Editors, published by John Wiley & Sons, Inc., 1975.

The term "solid dispersion" in the sense of the invention is defined as a system in a solid state (as opposed to a liquid or gaseous state) comprising at least three components, wherein at least one component or a combination of compounds is dispersed evenly throughout the at least other two components. For example, one or more active ingredients or combinations of active ingredients is dispersed in a matrix comprised of the pharmaceutically acceptable water-soluble polymers and the optional at least one a pharmaceutically acceptable surfactant.

The term "solid dispersion" encompasses systems having small particles, typically of less than e.g. 1 pm in diameter, of one phase dispersed in another phase. If said dispersion of components is such that the system is chemically and physically uniform or homogenous throughout or consists of one phase (as defined in thermodynamics), said solid dispersion is defined as "solid solution" or a "glassy solution".

A glassy solution is a homogeneous, glassy system wherein a solute is dissolved in a glassy solvent. Glassy solutions and solid solutions of said at least one HIV protease inhibitor are preferred physical systems. The invention systems in general are supposed to not contain significant amounts of active ingredient(s) in crystalline or microcrystalline state.

For the purpose of the present invention the at least one HIV protease inhibitor is selected from the group consisting of but not limited to: ritonavir, lopinavir, indinavir, saquinavir, amprenavir, nelfinavir, atazanavir, tipranavir, palinavir, fosemprenavir, darunavir, and combinations thereof, whereas ritonavir is the most preferred HIV protease inhibitor to be employed in the present invention.

In on embodiment of the invention the HIV protease inhibitor is a combination of two or more HIV protease inhibitors. In particular, the invention encompasses the combination of two, three, four, five or six HIV protease inhibitors. However, the invention is not limited to this number of HIV protease inhibitors and combinations of more than six HIV protease inhibitors are encompassed by the present invention as well. The combination of ritonavir and lopinavir as HIV protease inhibitors which may be formulated into the dosage form is preferred.

In a preferred embodiment of the invention, the pharmaceutical dosage form comprises a combination of ritonavir and lopinavir as HIV protease inhibitors, wherein the weight ratio of ritonavir and lopinavir ranges from 1:10 to 10:1. In particular, the weight ratio of ritonavir and lopinavir is 1:4

The pharmaceutical dosage form of the invention comprises 2 to 40%, preferably 10 to 40% by weight of one HIV protease inhibitor or of a combination of two or more HIV protease inhibitors as defined above.

In one embodiment, the polymer (B) is selected from the group consisting of polyethylene oxides, polyvinylacetate polyvinylpyrrolidone copolymers, polyethylene glycol polyvinylalcohol copolymers, methacrylic acid copolymers, aminoalkyl methacrylate copolymers, carboxylic acid functionalized polymethacrylates, amine-functionalized polymethacrylates, poly(vinyl acetal) diethylaminoacetate, polyvinyl alcohol/polyvinyl acetate copolymers and combinations thereof with the proviso that the polymer (B) has a Tg of less than 50 °C, preferably less than 40°C.

In a preferred embodiment of the invention the polymer (B) is polyethylene glycol (PEG), in particular PEG having an average molecular weight (MW) of between 1500 and 200000, preferably of between 4000 and 8000, more preferably of 6000.

The pharmaceutical dosage form comprises from 10 to 50% by weight, preferably from 10 to 40% by weight of the polymer (B).

The polymer (A) suitable for use with the present invention is selected from the group consisting of of carboxymethyl cellulose, sodium salt, polyvinyl alcohol, hydroxyethyl cellulose, polyvinylpyrrolidone vinylacetate copypolymer 6:4, polyvinyl caprolactam-polyvinyl acetate-polyethylene glycol graft copolymer, methyl celluloses, hydroxylpropyl cellulose, low substituted hydroxypropyl cellulose, methacrylic acid copolymers, aminoalkyl methacrylate copolymers, carboxylic acid functionalized polymethacrylates, amine-functionalized polymethacrylates, poly(vinyl acetal) diethylaminoacetate, hydroxypropyl methylcellulose phthalate and combinations thereof. The selected polymer (A) has a Tg of at least 50 °C, preferably of between 50°C and 250°C.

The pharmaceutical dosage form comprises from 20 to 49% by weight, preferably from 30 to 49%, 30 to 48%, 30 to 47%, 30 to 46% or 30 to 45% by weight of the polymer (A).

In one embodiment of the invention, the polymer (A) and the polymer (B) together are present in an amount of 50 to 85% by weight, preferably 50 to 70% by weight of the pharmaceutical dosage form.

The pharmaceutical dosage form of the present invention optionally comprises at least one pharmaceutically acceptable surfactant.

The surfactant is advantageous to enhance both wettability and processability of the polymers in use. Using neither surfactant nor plasticizer, the manufacturability (e.g. by hot melt extrusion) would be poor, meaning that the resulting dry granules or extrudate will be brittle. Moreover, the melting point of the active ingredient-excipients mixture (e.g. granules, extrudate, or physical mixture) would be significantly higher than without the use of surfactant or plasticizer. This would lead primarily to increased energy costs for the manufacturing process, but secondarily, in many cases the higher process temperature needed for melting of the mass will lead to degradation of the active pharmaceutical ingredient. Finally, use of surfactants is beneficial in view of the contact angle for liquids - for both in-vitro dissolution testing of dosage forms and bodily fluids in the patient: Many active pharmaceutical ingredients but also many inactive pharmaceutical ingredients suffer from poor wettability which can lead to poor reproducibility of drug release or even undesirable, non-reproducible sustained release. Surfactants lower the surface tension and allow for liquids to improve the moistening of those problematic substances resulting in higher bioavailability, enhanced (fast) release and thus, improved clinical effectiveness and early onset of clinical effect.

The present invention encompasses said at least one pharmaceutically acceptable surfactant being selected from the group consisting of sorbitan laurates, sorbitan oleates, sorbitan sesquioleate, polyethylene glycol 20 sorbitan monooleate, polyethylene glycol 80 sorbitan monolaurate, polyethylene glycol 20 sorbitan trioleate, polyethylene glycol sorbitan hexaoleate, sodium dodecyl sulfate, polyoxyl 35 (hydrogenated) castor oil, polyoxyl 40 (hydrogenated) castor oil, polyethylene glycol castor wax, polyethylene glycol sorbitan trioleate, triisostearin polyethylene glycol 6 esters, triglycerides of caprylic/capric acid, monoglycerides or diglycerides of caprylic/capric acid in glycerol, caprylic/capric acid partial glyceride-6 ethyleneoxide, propylene glycol and mono- or dicaprylate, glyceryl tricaprylate/caprate, polyethylene glycol 2 oleate, polyethylene glycol 4 laurate, polyethylene glycol 10 laurate (sodium), polyethylene glycol 20 isohexadecanoate, copolymers of propylene oxide and ethylene oxide, polypropylene glycol 15 stearate, polypropylene glycol 15 stearyl alcohol, unsaturated polyglycolyzed glycerides, glyceryl monolinoleate, decaglyceryl decaoleate, propylene glycol laurate, glyceryl and polyethylene glycol esters, oleoyl polyoxylglycerides, triglyceryl monooleate, glyceryl monooleate, sorbitan monolaurate, sorbitan monooleate, sorbitan dioleate, hexaglyceryl dioleate, polyglyceryl 4 oleate, polyglycolyzed glycerides, saturated C8-C10 polyglycolized glycerides, palm kemelamide, polysorbate 80, sucrose laurate, quaternary ammonium salt, distilled acetylated monoglycerides, distilled acetylated monoglycerides, sesame oil, olive oil, oleyl alcohol, alcohol ethoxylate, poloxamer 188, poloxamer 407 and combinations thereof.

In one embodiment the surfactant is a combination of two or more, preferably a combination of two, three, four, five or six pharmaceutically acceptable surfactants each of which having an HLB value from 4 to 10.

In an alternative embodiment the surfactant is a combination of at least two pharmaceutically acceptable surfactants, preferably a combination of two, three, four, five or six pharmaceutically acceptable surfactants, wherein at least one surfactant of said combination has an HLB value of less than 4 or more than 10 and at least one further surfactant of said combination has an HLB value of 4 to 10.

In each case the selection of surfactants is not limited to the number of surfactants, and combinations of more than six pharmaceutically acceptable surfactants are encompassed by the present invention as well.

The term "HLB value" as used herein refers the hydrophilic lipophilic balance (HLB) value. The HLB system (Fiedler, H. B. , Encylopedia of Excipients, 5th ed., Aulendorf: ECV-Editio-Cantor-Verlag (2002)) attributes numeric values to surfactants, with lipophilic substances receiving lower HLB values und hydrophilic substances receiving higher HLB values. Surfactants having an HLB value of from 4 to 10 are in particular suitable for use in the present invention.

Preferably the term "pharmaceutically acceptable surfactant" as used herein refers to a pharmaceutically acceptable non-ionic surfactant.

It is preferred that the at least one pharmaceutically acceptable surfactant is present in the pharmaceutical dosage form in an amount of 1 to 15% by weight of the total dosage form, preferably 5 to 15% by weight of the total dosage form.

In a further embodiment of the invention the dosage form additionally comprises at least one pharmaceutically acceptable plasticizer.

As used herein, the term "plasticizers" refers to additives that increase the plasticity, fluidity and/or decrease the melting point of the polymer(s) used with the present invention.

The pharmaceutically acceptable plasticizer as employed in the present invention has an HLB value from 2 to 12, and is preferably selected from the group consisting of triethyl citrate, triacetin, dibutyl phthalate, diethyl phthalate, dibutyl sebacate, diethyl sebacate and propylene glycol and combinations thereof.

In an embodiment of the invention the pharmaceutical dosage form comprises from 1 to 20%, preferably from 3 to 15% by weight of said pharmaceutically acceptable plasticizer.

A preferred embodiment of the pharmaceutical dosage form suitable for oral administration comprises a solid dispersion of
(i) 10 to 40% by weight of the dosage form of at least one HIV protease inhibitor as described above;
(ii) 30 to 45% by weight of the dosage form of at least one first pharmaceutically acceptable water-soluble polymer (A) as described above, i.e., having a glass transition temperature (Tg) of at least 50°C and being not PVP or HPMC;
(iii) 10 to 40% by weight of the dosage form of at least one further pharmaceutically acceptable water-soluble polymer (B) as described above, i.e., having a glass transition temperature (Tg) of less than 50°C, preferably of less than 40°C, and
(iv) 5 to 15% by weight of the dosage form of at least one pharmaceutically acceptable surfactant as defined above. Combinations of two, three, four, five, six or more pharmaceutically acceptable surfactants as defined above, each of which having an HLB value from 4 to 10, or combinations of two, three, four, five, six or more surfactants, wherein at least one surfactant of said combination has an HLB value of less than 4 or more than 10 and at least one further surfactant of said combination has an HLB value of 4 to 10, are preferred.

Polymer (A) and polymer (B) together are present in an amount of 50 to 70% by weight, relative to the weight of the dosage form, provided that the sum of the percentages of (i) to (iv) does not exceed 100%.

A preferred embodiment of the invention provides a pharmaceutical dosage form suitable for oral administration comprising a solid dispersion of
(i) 10 to 30% by weight lopinavir and 3 to 6% by weight ritonavir, relative to the weight of the dosage form,
(ii) 30 to 45% by weight copovidone, relative to the weight of the dosage form,
(iii) 15 to 40% by weight PEG having an average MW of 4000 to 8000, preferable 6000, relative to the weight of the dosage form, and
(iv) 5 to 10% by weight, relative to the weight of the dosage form, of a combination of two, three, four, five, six or more, preferably two surfactants as defined above. Combinations of two, three, four, five, six or more, preferably two pharmaceutically acceptable surfactants each of which having an HLB value from 4 to 10, or combinations of two, three, four, five, six or more, preferably two surfactants, wherein at least one surfactant of said combination has an HLB value of less than 4 or more than 10 and at least one further surfactant of said combination has an HLB value of 4 to 10, are preferred.

In the embodiment copovidone and PEG together are present in an amount of 50 to 70% by weight, relative to the weight of the dosage form, provided that the sum of the percentages of (i) to (iv) does not exceed 100%.

In one embodiment of the aforementioned preferred embodiment, said pharmaceutical dosage form comprises a solid dispersion of lopinavir and ritonavir wherein the weight ratio of ritonavir to lopinavir is 1:4.

In a further embodiment of said aforementioned embodiment of the pharmaceutical dosage form the solid dispersion additionally comprises
(v) 3 to 15% by weight, relative to the weight of the dosage form, of at least one pharmaceutically acceptable plasticizer selected from the group consisting of triethyl citrate, triacetin, dibutyl phthalate, diethyl phthalate, dibutyl sebacate, diethyl sebacate and propylene glycol and combinations thereof.

The dosage forms of the invention may additionally comprise at least one additive selected from flow regulators, disintegrants, bulking agents (fillers) and lubricants. The at least one additive is present in an amount from about 0% to about 40% by weight, preferably from about 1 % to about 25% by weight, more preferably from about 1.5% to about 15% by weight, relative to the weight of the dosage form.

The dosage forms according to the invention are characterized by an excellent stability and, in particular, exhibit high resistance against recrystallization or decomposition of the active ingredient(s).

Particularly, the dosage forms according to the invention show improved bioavailability compared to conventional solid oral dosage forms such as physical powder mixtures, direct compressed tablets, wet or fluidized-bed granules for the use in tablets or capsules. This advantage is crucial for clinical effectiveness in the patient as it raises the bioavailability, particularly of ritonavir, which acts as a CYP3A4-inhibiting agent, which is inevitably necessary to raise the plasma levels of lopinavir to exceed the lower limit of clinical effectiveness of lopinavir.

This beneficial effect of the dosage forms according to the invention is caused by the hydrophilic nature of the polymers in use in combination with the use of surfactants and plasticizers as well as the use of a suitable process for production of the solid dispersions according to the invention. Such a suitable process can for example be hot melt extrusion. Hot melt extrusion is advantageous for use as a suitable process for manufacturing of the solid dispersions according to the invention because throughout recent years it has become a robust, reproducible process for manufacturing of solid pharmaceutical dosage forms. Although not being as widespread in use for manufacturing of pharmaceuticals as conventional manufacturing methods for pharmaceuticals, for the existing marketed products where it is already used, it could be shown that its use is economically worthwhile and the pharmaceutical intermediates (or resulting dosage forms respectively) that have been produced using it, have many positive features: Improvement of bioavailability when used with suitable excipients, high homogeneity when used in conjunction with rather low dosed active pharmaceutical ingredients (below 50% by total mass of the individual dosage form), good microbial, chemical and physical stability of the intermediates and finished dosage forms.

The combination of water-soluble polymers (A) and (B) as defined herein allow for the preparation of solid dispersions that are mechanically stable and, within ordinary temperature ranges, sufficiently temperature stable so that the solid dispersions may be used as dosage forms without further processing or be compacted to tablets.

Various methods can be used for manufacturing the solid dosage forms according to the invention as outlined e. g. in W02005/039551.

It is an objective of the invention to provide the pharmaceutical dosage as described herein for use in medicine. The pharmaceutical dosage form of the invention is in particular useful for the treatment of HIV infection in a mammal.

Exemplary compositions of the present invention for combined administration of one or more HIV protease inhibitors are given in Tables 1 to 3 below.

**Table 1.**

| Component | % By Weight |
|---|---|
| Lopinavir | 20.0 |
| Ritonavir | 5.0 |
| PEG 6000 | 23.5 |
| Copovidone | 40.0 |
| Triethyl citrate | 5.0 |
| Sodium lauryl sulfate | 5.0 |
| Silicon dioxide | 0.5 |
| Sodium stearyl fumarate | 1.0 |

**Table 2.**

| Component | % By Weight |
|---|---|
| Lopinavir | 20.0 |
| Ritonavir | 5.0 |
| PEG 6000 | 33.8 |
| Copovidone | 30.0 |
| Dibutylsebacat | 3.0 |
| Macrogol-glycerol hydroxystearate 40 | 7.0 |
| Silicon dioxide | 0.7 |
| Sodium stearyl fumarate | 0.5 |

**Table 3.**

| Component | % By Weight |
|---|---|
| Lopinavir | 20.0 |
| Ritonavir | 5.0 |
| PEG 6000 | 20.5 |
| Copovidone | 37.5 |
| Triethyl citrate | 7.0 |
| Sorbitan laurate | 7.0 |
| Silicon dioxide | 1.0 |
| Sodium stearyl fumarate | 2.0 |

The above compositions may be processed e. g. by melt extrusion. The resulting extrudates may be used as such or milled and compressed into tablets, preferably by the use of suitable tabletting aidswell known in the art.

The following examples will further illustrate the invention without limiting it.

### Comparative examples

### Example 1

A mixture of copovidone (N-vinyl pyrrolidone/vinyl acetate copolymer 60: 40; 40 % by weight of the dosage form) as first water-soluble polymer having a Tg of at least 50°C and PEG 6000 (polyethylene glycol, MW 6000; 23.5 % by weight of the dosage form) as second water-soluble polymer having a Tg of less than 50°C was mixed with ritonavir (5 % by weight of the dosage form) and lopinavir (20 % by weight of the dosage form). Triethyl citrate (5 % by weight of the dosage form) was used as plasticizer, and sodium lauryl sulfate (5 % by weight of the dosage form) as surfactant. Silicon dioxide (0.5 % by weight of the dosage form) was employed as flow agent, while sodium stearyl fumarate (1.0 % by weight of the dosage form) was used as lubricant for forming the dosage form.

The mixture was processed as well known in the art for providing a dosage form suitable for medical use.

### Example 2

A mixture of copovidone (N-vinyl pyrrolidone/vinyl acetate copolymer 60: 40; 30 % by weight of the dosage form) as first water-soluble polymer having a Tg of at least 50°C and PEG 6000 (polyethylene glycol, MW 6000; 33.8 % by weight of the dosage form) as second water-soluble polymer having a Tg of less than 50°C was mixed with ritonavir (5 % by weight of the dosage form) and lopinavir (20 % by weight of the dosage form). Dibutylsebacat (3.0 % by weight of the dosage form) was used as plasticizer while macrogol-glycerol hydroxystearate 40 (7.0 % by weight of the dosage form) was employed as surfactant. Silicon dioxide (0.7 % by weight of the dosage form) was employed as flow agent, while sodium stearyl fumarate (0.5 % by weight of the dosage form) was used as lubricant for forming the dosage form.

The mixture was processed as well known in the art for providing a dosage form suitable for medical use.

### Example 3

A mixture of copovidone (N-vinyl pyrrolidone/vinyl acetate copolymer 60: 40; 37.5 % by weight of the dosage form) as first water-soluble polymer having a Tg of at least 50°C and PEG 6000 (polyethylene glycol, MW 6000; 20.5 % by weight of the dosage form) as second water-soluble polymer having a Tg of less than 50°C was mixed with ritonavir (5 % by weight of the dosage form) and lopinavir (20 % by weight of the dosage form). Triethyl citrate (7.0 % by weight of the dosage form) was used as plasticizer while sorbitan laurate (7.0 % by weight of the dosage form) was employed as surfactant. Silicon dioxide (1.0 % by weight of the dosage form) was employed as flow agent, while sodium stearyl fumarate (2.0 % by weight of the dosage form) used as lubricant when forming the dosage form.

The mixture was processed as well known in the art for providing a dosage form suitable for medical use.

The following items are part of the description of the invention.
Item 1. A pharmaceutical dosage form suitable for oral administration comprising a solid dispersion of
   (i) at least one HIV protease inhibitor,
   (ii) at least one first pharmaceutically acceptable water-soluble polymer (A),
   (iii) at least one second pharmaceutically acceptable water-soluble polymer (B), and
   (iv) optionally at least one pharmaceutically acceptable surfactant,
   wherein the at least one first pharmaceutically acceptable water-soluble polymer (A)
   a) has a glass transition temperature (Tg) of at least 50°C and
   b) is present in an amount of 10% to 49% by weight of the dosage form
      and
   c) is not polyvinylpyrrolidone (PVP) or hydroxypropyl methylcellulose (HPMC), and
   wherein the at least one second pharmaceutically acceptable water-soluble polymer (B) has a Tg of less than 50°C.
Item 2. The pharmaceutical dosage form of item 1 comprising a glassy or solid solution of said at least one HIV protease inhibitor.
Item 3. The pharmaceutical dosage form of item 1 or 2, wherein the at least one HIV protease inhibitor is selected from the group consisting of: Ritonavir, Lopinavir, Indinavir, Saquinavir, Amprenavir, Nelfinavir, Atazanavir, Tipranavir, Palinavir, Fosamprenavir, Darunavir, preferably Ritonavir and combinations thereof.
Item 4. The pharmaceutical dosage form of any one of items 1 to 3, wherein the at least one HIV protease inhibitor is a combination of two, three, four, five or six HIV protease inhibitors, preferably a combination of ritonavir and lopinavir.
Item 5. A pharmaceutical dosage form of item 4, wherein the at least one HIV protease inhibitor is a combination of ritonavir and lopinavir having a weight ratio from 1:10 to 10:1.
Item 6. The pharmaceutical dosage form of item 5, wherein the weight ratio of ritonavir and lopinavir is 1:4.
Item 7. The pharmaceutical dosage form of any one of items 1 to 6, comprising 2-40% by weight, preferably 10% to 40% by weight of the at least one HIV protease inhibitor.
Item 8. The pharmaceutical dosage form of any one of items 1 to 7, wherein the polymer (B) has a Tg of less than 40°C.
Item 9. The pharmaceutical dosage form of any one of items 1 to 8, wherein the polymer (B) is selected from the group consisting of polyethylene oxides/polyethylene glycols, polyvinylacetate polyvinylpyrrolidone copolymers, polyethylene glycol polyvinylalcohol copolymers, methacrylic acid copolymers, aminoalkyl methacrylate copolymers, carboxylic acid functionalized polymethacrylates, amine-functionalized polymethacrylates, poly(vinyl acetal) diethylaminoacetate, polyvinyl alcohol/polyvinyl acetate copolymers and combinations thereof, and wherein the polymer (B) has a Tg of less than 50°C, preferably less than 40°C.
Item 10. The pharmaceutical dosage form of item 9, wherein the polymer (B) is polyethylene glycol (PEG), preferably PEG having an average MW of between 1500 and 200000, preferably of between 4000 and 8000, more preferably of 6000.
Item 11. The pharmaceutical dosage form of any one of items 1 to 10, wherein the polymer (B) is present in an amount of 10% to 50% by weight, preferably 10% to 40% by weight, relative to the weight of the dosage form.
Item 12. The pharmaceutical dosage form of any one of items 1 to 11, wherein the at least one first water-soluble polymer (A) has a Tg of about 50°C to about 250°C, preferably of about 80°C to about 225°C.
Item 13. The pharmaceutical dosage form of any one of items 1 to 12, wherein the polymer (A) is selected from the group consisting of carboxymethyl cellulose, sodium salt, polyvinyl alcohol, hydroxyethyl cellulose, polyvinylpyrrolidone vinylacetate copypolymer 6:4, polyvinyl caprolactam - polyvinyl acetate - polyethylene glycol graft copolymer, methyl celluloses, hydroxylpropyl cellulose, low substituted hydroxypropyl cellulose, methacrylic acid copolymers, aminoalkyl methacrylate copolymers, carboxylic acid functionalized polymethacrylates, amine-functionalized polymethacrylates, poly(vinyl acetal) diethylaminoacetate, hydroxypropyl methylcellulose phthalate and combinations thereof, and
wherein the polymer (A) has a Tg of at least 50°C, preferably between 50°C and 250°C.
Item 14. The pharmaceutical dosage form of any one of items 1 to 13, wherein the polymer (A) is present in an amount of 20% to 49% by weight, preferably 30% to 49%, 30% to 48%, 30% to 47%, 30% to 46% or 30% to 45% by weight, relative to the weight of the dosage form.
Item 15. The pharmaceutical dosage form of any one of items 1 to 14, wherein the polymer (A) and the polymer (B) together are present in an amount of 50% to 85% by weight, preferably 50% to 70% by weight, relative to the weight of the dosage form.
Item 16. The pharmaceutical dosage form of any one of items 1 to 15, wherein the at least one pharmaceutically acceptable surfactant is selected from the group consisting of sorbitan laurates, sorbitan oleates, sorbitan sesquioleate, polyethylene glycol 20 sorbitan monooleate, polyethylene glycol 80 sorbitan monolaurate, polyethylene glycol 20 sorbitan trioleate, polyethylene glycol sorbitan hexaoleate, sodium dodecyl sulfate, polyoxyl 35 (hydrogenated) castor oil, polyoxyl 40 (hydrogenated) castor oil, polyethylene glycol castor wax, polyethylene glycol sorbitan trioleate, triisostearin polyethylene glycol 6 esters, triglycerides of caprylic/capric acid, monoglycerides or diglycerides of caprylic/capric acid in glycerol, caprylic/capric acid partial glyceride-6 ethyleneoxide, propylene glycol and mono- or dicaprylate, glyceryl tricaprylate/caprate, polyethylene glycol 2 oleate, polyethylene glycol 4 laurate, polyethylene glycol 10 laurate (Sodium), polyethylene glycol 20 isohexadecanoate, copolymers of propylene oxide and ethylene oxide, polypropylene glycol 15 stearate, polypropylene glycol 15 stearyl alcohol, unsaturated polyglycolyzed glycerides, glyceryl monolinoleate, decaglyceryl decaoleate, propylene glycol laurate, glyceryl and polyethylene glycol esters, oleoyl polyoxylglycerides, triglyceryl monooleate, glyceryl monooleate, sorbitan monolaurate, sorbitan monooleate, sorbitan dioleate, hexaglyceryl dioleate, polyglyceryl 4 oleate, polyglycolyzed glycerides, saturated C8-C10 polyglycolized glycerides, palm kemelamide, polysorbate 80, sucrose laurate, quaternary ammonium salt, distillated acetylated monoglycerides, distilled acetylated monoglycerides, sesame oil, olive oil, oleyl alcohol, alcohol ethoxylate, poloxamer 188, poloxamer 407 and combinations thereof.
Item 17. The pharmaceutical dosage form of any one of items 1 to 16, wherein the at least one pharmaceutically acceptable surfactant is a combination of at least two surfactants, preferably a combination of two, three, four, five or six surfactants, each surfactant having an HLB value from 4 to 10.
Item 18. The pharmaceutical dosage form of any one of items 1 to 16, wherein the at least one pharmaceutically acceptable surfactant is a combination of at least two surfactants, preferably a combination of two, three, four, five or six surfactants, wherein at least one surfactant of said combination has an HLB value of less than 4 or more than 10, and the at least other surfactant of said combination has an HLB value of 4 to 10.
Item 19. The pharmaceutical dosage form of any one of items 1 to 18, wherein the at least one pharmaceutically acceptable surfactant is present in an amount of 1% to 15% by weight, preferably 5% to 15% by weight, relative to the weight of the dosage form.
Item 20. The pharmaceutical dosage form of any one of items 1 to 19, the solid dispersion further comprising
   (v) at least one pharmaceutically acceptable plasticizer.
Item 21. The pharmaceutical dosage form of item 20, wherein the at least one pharmaceutically acceptable plasticizer has an HLB value from 2 to 12 and is preferably selected from the group consisting of triethyl citrate, triacetin, dibutyl phthalate, diethyl phthalate, dibutyl sebacate, diethyl sebacate, propylene glycol and combinations thereof.
Item 22. The pharmaceutical dosage form of item 20 or 21, wherein the plasticizer is present an amount of 1% to 20% by weight, preferably 3% to 15% by weight, relative to the weight of the dosage form.
Item 23. A pharmaceutical dosage form suitable for oral administration comprising a solid dispersion of
   (i) 10% to 40% by weight of at least one HIV protease inhibitor as defined in any one of items 1 to 7, relative to the weight of the dosage form,
   (ii) 30% to 45% by weight of at least one pharmaceutically acceptable water-soluble polymer (A) as defined in any one of items 1 and 13, relative to the weight of the dosage form,
   (iii) 10% to 40% by weight of at least one pharmaceutically acceptable water-soluble polymer (B) as defined in any one of items 1 and 9 to 11, relative to the weight of the dosage form,
   (iv) 5% to 15% by weight of at least one pharmaceutically acceptable surfactant as defined in any one of items 16 to 18, relative to the weight of the dosage form,
      wherein the polymer (A) and the polymer (B) together are present in an amount of 50% to 70% by weight, relative to the weight of the dosage form,
      provided that the sum of the percentages of (i) to (iv) does not exceed 100%.
Item 24. A pharmaceutical dosage form suitable for oral administration
   comprising a solid dispersion of
   (i) 10% to 30% by weight Lopinavir and 3% to 6% by weight ritonavir, relative to the weight of the dosage form,
   (ii) 30% to 45% by weight copovidone, relative to the weight of the dosage form,
   (iii) 15% to 40% by weight PEG having an average MW of 4000 to 8000, preferably of 6000, relative to the weight of the dosage form,
   (iv) 5% to 10% by weight of a combination of two surfactants as defined in items 16 to 18, relative to the weight of the dosage form,
wherein copovidone and PEG together are present in an amount of 50% to 70% by weight, relative to the weight of the dosage form, provided that the sum of the percentages of (i) to (iv) does not exceed 100%.
Item 25. The pharmaceutical dosage form of item 24, wherein the weight ratio of ritonavir and lopinavir is 1:4.
Item 26. The pharmaceutical dosage form of item 24 or 25, the solid dispersion further comprising
   (v) 3% to 15% by weight, relative to the weight of the dosage form, of at least one pharmaceutically acceptable plasticizer as defined in item 21, provided that the sum of the percentages of (i) to (v) does not exceed 100%.
Item 27. The pharmaceutical dosage form of any one of items 1 to 26 further comprising at least one additive selected from flow regulators, disintegrants, bulking agents and lubricants.
Item 28. The pharmaceutical dosage form of item 27 wherein the at least one additive is present in an amount from of 0% to about 40% by weight, preferably from of 1 % to about 25% by weight, most preferably from of 1.5% to about 15% by weight, relative to the weight of the dosage form.
Item 29. The pharmaceutical dosage form of any one of items 1 to 28 for use in medicine, in particular for the treatment of HIV infection in a mammal.

## Claims

1. A pharmaceutical dosage form suitable for oral administration comprising a solid dispersion of
(i) at least one HIV protease inhibitor,
(ii) at least one first pharmaceutically acceptable water-soluble polymer (A),
(iii) at least one second pharmaceutically acceptable water-soluble polymer (B), and
(iv) optionally at least one pharmaceutically acceptable surfactant,
wherein the at least one first pharmaceutically acceptable water-soluble polymer (A)
a) has a glass transition temperature (Tg) of at least 50°C and
b) is present in an amount of 10% to 49% by weight of the dosage form
and
c) is not polyvinylpyrrolidone (PVP) or hydroxypropyl methylcellulose (HPMC), and
wherein the at least one second pharmaceutically acceptable water-soluble polymer (B) has a Tg of less than 50°C.

2. The pharmaceutical dosage form of claim 1, wherein the at least one HIV protease inhibitor is selected from the group consisting of: Ritonavir, Lopinavir, Indinavir, Saquinavir, Amprenavir, Nelfinavir, Atazanavir, Tipranavir, Palinavir, Fosamprenavir, Darunavir, preferably Ritonavir and combinations thereof.

3. The pharmaceutical dosage form of claim 1 or 2, wherein the at least one HIV protease inhibitor is a combination of two, three, four, five or six HIV protease inhibitors, preferably a combination of ritonavir and lopinavir.

4. The pharmaceutical dosage form of any one of claims 1 to 3, comprising 2-40% by weight, preferably 10% to 40% by weight of the at least one HIV protease inhibitor.

5. The pharmaceutical dosage form of any one of claims 1 to 4, wherein the polymer (B) is selected from the group consisting of polyethylene oxides/polyethylene glycols, polyvinylacetate polyvinylpyrrolidone copolymers, polyethylene glycol polyvinylalcohol copolymers, methacrylic acid copolymers, aminoalkyl methacrylate copolymers, carboxylic acid functionalized polymethacrylates, amine-functionalized polymethacrylates, poly(vinyl acetal) diethylaminoacetate, polyvinyl alcohol/polyvinyl acetate copolymers and combinations thereof, and
wherein the polymer (B) has a Tg of less than 50 °C, preferably less than 40°C.

6. The pharmaceutical dosage form of claim 5, wherein the polymer (B) is polyethylene glycol (PEG), preferably PEG having an average MW of between 1500 and 200000, or of between 4000 and 8000, more preferably of 6000.

7. The pharmaceutical dosage form of any one of claims 1 to 6, wherein the polymer (B) is present in an amount of 10% to 50% by weight, preferably 10% to 40% by weight, relative to the weight of the dosage form.

8. The pharmaceutical dosage form of any one of claims 1 to 7, wherein the at least one first water-soluble polymer (A) has a Tg of 50°C to 250°C, preferably of 80°C to 225°C.

9. The pharmaceutical dosage form of any one of claims 1 to 8, wherein the polymer (A) is selected from the group consisting of carboxymethyl cellulose, sodium salt, polyvinyl alcohol, hydroxyethyl cellulose, polyvinylpyrrolidone vinylacetate copypolymer 6:4, polyvinyl caprolactam - polyvinyl acetate - polyethylene glycol graft copolymer, methyl celluloses, hydroxylpropyl cellulose, low substituted hydroxypropyl cellulose, methacrylic acid copolymers, aminoalkyl methacrylate copolymers, carboxylic acid functionalized polymethacrylates, amine-functionalized polymethacrylates, poly(vinyl acetal) diethylaminoacetate, hydroxypropyl methylcellulose phthalate and combinations thereof, and
wherein the polymer (A) has a Tg of at least 50°C, preferably between 50°C and 250°C.

10. The pharmaceutical dosage form of any one of claims 1 to 9, wherein the polymer (A) is present in an amount of 20% to 49% by weight, preferably 30% to 49%, 30% to 48%, 30% to 47%, 30% to 46% or 30% to 45% by weight, relative to the weight of the dosage form.

11. The pharmaceutical dosage form of any one of claims 1 to 10, wherein the polymer (A) and the polymer (B) together are present in an amount of 50% to 85% by weight, preferably 50% to 70% by weight, relative to the weight of the dosage form.

12. The pharmaceutical dosage form of any one of claims 1 to 11, wherein the at least one pharmaceutically acceptable surfactant is selected from the group consisting of sorbitan laurates, sorbitan oleates, sorbitan sesquioleate, polyethylene glycol 20 sorbitan monooleate, polyethylene glycol 80 sorbitan monolaurate, polyethylene glycol 20 sorbitan trioleate, polyethylene glycol sorbitan hexaoleate, sodium dodecyl sulfate, polyoxyl 35 (hydrogenated) castor oil, polyoxyl 40 (hydrogenated) castor oil, polyethylene glycol castor wax, polyethylene glycol sorbitan trioleate, triisostearin polyethylene glycol 6 esters, triglycerides of caprylic/capric acid, monoglycerides or diglycerides of caprylic/capric acid in glycerol, caprylic/capric acid partial glyceride-6 ethyleneoxide, propylene glycol and mono- or dicaprylate, glyceryl tricaprylate/caprate, polyethylene glycol 2 oleate, polyethylene glycol 4 laurate, polyethylene glycol 10 laurate (Sodium), polyethylene glycol 20 isohexadecanoate, copolymers of propylene oxide and ethylene oxide, polypropylene glycol 15 stearate, polypropylene glycol 15 stearyl alcohol, unsaturated polyglycolyzed glycerides, glyceryl monolinoleate, decaglyceryl decaoleate, propylene glycol laurate, glyceryl and polyethylene glycol esters, oleoyl polyoxylglycerides, triglyceryl monooleate, glyceryl monooleate, sorbitan monolaurate, sorbitan monooleate, sorbitan dioleate, hexaglyceryl dioleate, polyglyceryl 4 oleate, polyglycolyzed glycerides, saturated C8-C10 polyglycolized glycerides, palm kemelamide, polysorbate 80, sucrose laurate, quaternary ammonium salt, distillated acetylated monoglycerides, distilled acetylated monoglycerides, sesame oil, olive oil, oleyl alcohol, alcohol ethoxylate, poloxamer 188, poloxamer 407 and combinations thereof.

13. The pharmaceutical dosage form of any one of claims 1 to 12, wherein the at least one pharmaceutically acceptable surfactant is either
a) a combination of at least two surfactants, preferably a combination of two, three, four, five or six surfactants, each surfactant having an HLB value from 4 to 10, or
b) a combination of at least two surfactants, preferably a combination of two, three, four, five or six surfactants, wherein at least one surfactant of said combination has an HLB value of less than 4 or more than 10, and the at least other surfactant of said combination has an HLB value of 4 to 10.

14. The pharmaceutical dosage form of any one of claims 1 to 13, the solid dispersion further comprising
(v) at least one pharmaceutically acceptable plasticizer.

15. The pharmaceutical dosage form of claim 14, wherein the at least one pharmaceutically acceptable plasticizer has an HLB value from 2 to 12 and is preferably selected from the group consisting of triethyl citrate, triacetin, dibutyl phthalate, diethyl phthalate, dibutyl sebacate, diethyl sebacate, propylene glycol and combinations thereof.

16. A pharmaceutical dosage form suitable for oral administration comprising a solid dispersion of
(i) 10% to 40% by weight of at least one HIV protease inhibitor as defined in any one of claims 1 to 3, relative to the weight of the dosage form,
(ii) 30% to 45% by weight of at least one pharmaceutically acceptable water-soluble polymer (A) as defined in any one of claims 1, 8 and 9, relative to the weight of the dosage form,
(iii) 10% to 40% by weight of at least one pharmaceutically acceptable water-soluble polymer (B) as defined in any one of claims 1, 5 and 6, relative to the weight of the dosage form,
(iv) 5% to 15% by weight of at least one pharmaceutically acceptable surfactant as defined in any one of claims 12 and 13, relative to the weight of the dosage form,
wherein the polymer (A) and the polymer (B) together are present in an amount of 50% to 70% by weight, relative to the weight of the dosage form, provided that the sum of the percentages of (i) to (iv) does not exceed 100%.

17. A pharmaceutical dosage form suitable for oral administration comprising a solid dispersion of
(i) 10% to 30% by weight Lopinavir and 3% to 6% by weight ritonavir, relative to the weight of the dosage form,
(ii) 30% to 45% by weight copovidone, relative to the weight of the dosage form,
(iii) 15% to 40% by weight PEG having an average MW of 4000 to 8000, preferably of 6000, relative to the weight of the dosage form,
(iv) 5% to 10% by weight of a combination of two surfactants as defined in any one of claims 12 and 13, relative to the weight of the dosage form,
wherein copovidone and PEG together are present in an amount of 50% to 70% by weight, relative to the weight of the dosage form, provided that the sum of the percentages of (i) to (iv) does not exceed 100%.

18. The pharmaceutical dosage form of claim 17, wherein the weight ratio of ritonavir and lopinavir is 1:4.

19. The pharmaceutical dosage form of claim 17 or 18, the solid dispersion further comprising
(v) 3% to 15% by weight, relative to the weight of the dosage form, of at least one pharmaceutically acceptable plasticizer as defined in claim 15, provided that the sum of the percentages of (i) to (v) does not exceed 100%.

20. The pharmaceutical dosage form of any one of claims 1 to 19 for use in medicine, in particular for the treatment of HIV infection in a mammal.
